Europäisches Patentamt

European Patent Office

Office européen des brevets

(11) Publication number: **0 363 569**
**A1**

# (12) EUROPEAN PATENT APPLICATION

(21) Application number: **89111455.5**

(22) Date of filing: **23.06.89**

(51) Int. Cl.⁵: **A61K 7/50**

(30) Priority: **11.10.88 IT 2226588**

(43) Date of publication of application:
**18.04.90 Bulletin 90/16**

(84) Designated Contracting States:
**AT BE CH DE ES FR GB GR LI LU NL SE**

(71) Applicant: **COMPAGNA ITALIANA SALI C.I.S. S.p.A.**
**Via Stradonazzi 42**
**Donada (Rovigo)(IT)**

(72) Inventor: **Cibrario, Graziella**
**Via Cappellina 13**
**I-Vercelli(IT)**

(74) Representative: **Rapisardi, Mariacristina, Dr. Proc.**
**STUDIO TECNICO LEGALE RAPISARDI Largo V Alpini 15**
**I-20145 Milano(IT)**

(54) **Spontaneously water-dispersible composition particularly suitable for treating the skin.**

(57) The spontaneously water-dispersible composition, particularly suitable for treating the skin, comprises sodium chloride and an effervescent agent composed of sodium bicarbonate and tartaric acid.

EP 0 363 569 A1

# SPONTANEOUSLY WATER-DISPERSIBLE COMPOSITION PARTICULARLY SUITABLE FOR TREATING THE SKIN

This present invention relates to a spontaneously water-dispersible composition particularly suitable for treating the skin.

Besides having a hygienic washing function, the immersion of the human body in a tub containing water or under a shower is currently increasingly assuming the function of also giving the person a relaxing effect which reduces stress and sometimes stimulates skin metabolism in a natural manner.

It is by now generally known that the combined effect of the temperature of the water of the bath or shower, in combination with dermo-active substances and sometimes with substances having a pharmacological action, contributes to alleviate fatigue and leaves the skin soft, allowing the expulsion therefrom of toxins or impurities and most of all providing it with a protection against the calcareous substances contained in the water.

The purposes normally sought with these products are to formulate said products so as to address the benefits which derive therefrom to the various problems related to the health and beauty of the human body; for example, there are products which mainly tend to eliminate the impurities from the skin by means of mineral substances, as well as products used exclusively to relax or give tone to the user's physique.

The aim of the present invention is to provide a spontaneously water-dispersible composition, particularly suitable for treating the skin, which can substantially reproduce the same beneficial effects as seawater.

Within the scope of this aim, an important object of the invention is to provide a spontaneously water-dispersible composition, particularly suitable for treating the skin, which can be used indifferently for a bath, a shower and for local skin applications or for foot-baths, cold compresses, spongings, massages and the like.

Not least object of the present invention is to provide a spontaneously water-dispersible composition, particularly suitable for treating the skin, which gives the body of the user beneficial effects from the aesthetic point of view and from the cosmetic/curative point of view.

This aim, as well as these and other objects, are achieved by a spontaneously water-dispersible composition, particularly suitable for treating the skin, characterized in that it comprises sodium chloride and an effervescent agent.

According to a preferred embodiment of the invention, said effervescent agent is composed of sodium bicarbonate and tartaric acid and the weight ratio between sodium bicarbonate and tartaric acid is preferably comprised between 0.25 and 4.

According to another embodiment of the invention, the effervescent agent instead comprises glycine sodium carbonate and monobasic sodium citrate.

In this case the weight$ ratio between glycine sodium carbonate and monobasic sodium citrate is preferably comprised between 0.33 and 3.

The spontaneously dispersible composition according to the invention preferably furthermore comprises iodine contained as iodide or iodate, and the weight ratio between sodium chloride and iodine, as iodide or iodate, is preferably comprised between 10 and 100.

It has furthermore been observed that the beneficial effects of the invention are improved by the present of the so-called Spirulina alga, which is preferably contained in a weight percentage comprised between 1% and 55% with respect to the total weight of the composition.

According to a preferred embodiment of the invention, the composition furthermore comprises metallic ions; in particular it is possible to add one or more ions chosen among the following elements: iron, nickel, zinc, copper and manganese.

The iron ion is preferably comprised in an amount between 10 and 100 ppm in the finished product constituted by the composition ready for use.

The nickel ion is contained in an amount comprised between 10 to 100 ppm is the finished product.

The zinc ion is contained in an amount of 10-100 ppm in the finished product.

The copper ion is contained in an amount comprised between 10 and 100 ppm on the finished product.

The manganese ion is preferably contained in an amount comprised between 1 and 50 ppm in the finished product.

Scenting essences are furthermore preferably comprised in the composition, for example in an amount of 0.5%-10% of the weight of the finished product. The invention furthermore relates to the use of a composition as described above to prepare bath salts.

Further characteristics and advantages of the invention will become apparent from the description of some preferred but not exclusive embodiments of the composition, illustrated by way of non-limitative indication in the following examples.

## Example 1

Effervescent bath salts in powder form.

### Method of preparation

Direct mixing and packaging in a conditioned and dehumidified environment (RH < o = 40%; temperature < o = 25 degrees Centigrade).

### Formula of the components

1 - Sodium bicarbonate 5-60%
2 - Refined salt 10-55%
3 - Tartaric acid 5-60%
4 - Pulverized Spirulina alga 1-55%
5 - Scented essences 0.5-5%
6 - Potassium iodide 0,2-1%
7 - Tartrazine yellow dye (E102) 0.05-0.5%
8 - Patent blue dye (E131) 0.001-0.01%

### Mixture preparation process

The following are introduced in a powder mixer of appropriate capacity in this order: approximately two thirds of component 1, components 2 and 3, and approximately two thirds of component 4, forcing them through a 15-mesh screen.

The following are introduced in another powder mixer of adequate capacity, in this order: the remainder of components 1 and 4 and components 6, 8 and 9, forcing them through a 30-mesh screen; mixing is then performed for 5- 10 minutes.

Mixture "b" is added into the first mixer, and mixing is performed for 10-15 minutes; during the first 5 minutes, component 5 is sprayed onto the moving mass, with the aid of an airbrush, adjusting the pressure of the propellant fluid between 0.1 and 0.5 atm.

The mixture thus obtained may be divided into single or multiple-dose packages of material impermeable to water vapor.

## Example 2

Effervescent bath salts in small granules (20-30 mesh)

### Method of preparation

Granulation on a fluid bed, is a non-conditioned environment; packaging in a conditioned environment (R.H. < o = 65%; temperature < o = 25 degrees Centigrade).

### Formula of components

1 - Sodium bicarbonate 5-60%
2 - Refined salt 10-55%
3 - Purified tartaric acid 5-60%
4 - Pulverized Spirulina alga 1-55%
5 - Gelatine 1-5%
6 - Glycerine 0.1-0.5%
7 - Scented essences 0.5-5%
8 - Potassium iodate 0.2-1%
9 - Tartrazine yellow dye (E102) 0.05-0.5%
10 - Patent blue dye (E131) 0.001-0.01%

### Process for preparing the granulated product

Components 1, 2 and 4 are introduced in that order in a vertical fluid-bed granulator, forcing them through a 30-mesh screen; mixing is then performed for 3 minutes in a flow of warm air (50-60 degrees Centigrade) with minimum turbulence.

The granular solution is prepared by warm agitation (50-60 degrees Centigrade) with components 5, 6, 8, 9 and 10 in 8-10 parts (with respect to the total of the components to be dissolved) in aqua fontis.

The granulating solution is sprayed, at 1-2 atm of pressure, onto the mass, fluidified with water air (50-60 degrees Centigrade), in a condition of turbulence adequate to the operating requirements of the fluid bed.

Drying is performed in warm air (50-60 degrees Centigrade), with low turbulence, until the temperature of the air in output reaches 40-50 degrees Centigrade.

Component 3 is added, after forcing it through a 20-mesh screen, and mixing is performed for approximately 3 minutes in a flow of unheated air, with minimum turbulence, then component 7 is sprayed at a pressure of 2-3 atm, increasing turbulence according to operational requirements; mixing is finally performed for approximately 5 minutes with minimum turbulence.

The mixture thus obtained can be divided into single-or multiple-dose packages made of material impermeable to water vapor.

## Example 3

Effervescent bath salts, in medium grains (rice-grain size) and large grains (cherry-size)

### Method of preparation

Granulation (by extrusion) and packaging in an unconditioned environment, so long as it is protected from the direct action of weather.

Formula of the components

   1 - Glycine sodium carbonate 7.5-60%
   2 - Refined salt 10-55%
   3 - Monobasic sodium citrate 2.5-15%
   4 - Pulverized "Spirulina" alga 1-55%
   5 - Arabic gum 2-5%
   6 - Scented essences 1-2%
   7 - Potassium iodate 0.2-1%
   8 - Tartrazine yellow dye (E102) 0.05%-0.5%
   9 - Patent blue dye (E131) 0.001-0.01%

## Process for preparing the extruded granulated product

Components 1, 2, 3 and 4 are introduced in that order in a powder mixer of adequate capacity, forcing them through a 30-mesh screen, and mixing is performed for approximately 5 minutes.

A solution is prepared by heated agitation (50-60 degrees Centigrade), with components 5, 7, 8 and 9 in 2-3 pp (with respect to the total of the components to be dissolved) of aqua fontis; cooling to room temperature is allowed and component 6 is added while performing agitation.

Mixture "a" is mixed in the solution "b" for approximately 10 minutes and in any case until the dye is uniformly distributed.

The mixture is granulated with an extrusion granulator (of the scroll, oscillating or centrifugal type), equipped with a screen or perforated diaphragm adequate for the shape and dimensions of the granulated product to be obtained.

The product is dried in a forced-ventilation stove for 12-18 hours, at 35-40 degrees Centigrade, or in a fluid bed with low turbulence, with air at 40-50 degrees Centigrade, until the temperature of the outflowing air reaches 35 degrees Centigrade.

The mixture thus obtained may be divided into single-or multiple-dose packages made of material impermeable to water vapor.

## Example 4

Effervescent shower salts, in paste form

## Method of preparation

Mixing and packaging in an non-conditioned environment, so long as protected from the direct action of weather.

## Formula of the components

   1 - Glycine sodium carbonate 7.5-60%
   2 - Refined salt 10-55%
   3 - Monobasic sodium citrate 7.5-60%
   4 - Pulverized "Spirulina" alga 1-55%
   5 - Scented essences 0.5-5%
   6 - Potassium iodate 0.2-1%
   7 - Tartrazine yellow dye (E102) 0.05%-0.5%
   8 - Patent blue dye (E131) 0.001-0.01%
   9 - Glycerine 10-30%

## Paste preparation process

Components 1, 2, 3 and 4 are introduced in that order in a powder mixer of adequate capacity, and mixing is performed for approximately 5 minutes.

Mixture "a" is ground with a screenless pivot mill at high speed (15,000-20,000 rpm). The ground mixture is fed into a mixer-homogenizer for creams or ointments and mixing is performed with component 9, in which components 6, 7, and 8, finely pulverized, and component 5, as is, have been dispersed beforehand.

Mixing lasts 20-30 minutes and in any case last until the dye has completely and uniformly dispersed.

The mixture thus obtained can be divided into single-or multiple-dose packages made of material impermeable to water vapor.

## Example 5

Effervescent bath salts in tablet form

## 1 - Method of preparation in controlled environment

Direct mixing in conditioned environment (R.H. < 60%, temperature < 25 degrees Centigrade); compaction in a dehumidified environment (R.H. < 40%, temperature < 25 degrees Centigrade); packing in a conditioned environment (like mixing).

## Formula of the components

   1 - Sodium bicarbonate 5-60%
   2 - Mannitol 5-20%
   3 - Refined salt 10-35%
   4 - Tartaric acid 5-60%
   5 - Pulverized "Spirulina" alga 1-55%
   6 - Polyethyleneglycol 6000 0.5-4%
   7 - Scented essences 0.5-5%
   8 - Potassium iodide 0.2-1%
   9 - Tartrazine yellow dye (E102) 0.05%-0.5%
   10 - Patent blue dye (E131) 0.001-0.1%

## Process for preparing the mixture to be compacted

Approximately 2/3 of component 1, components 2-3-4 and approximately 2/3 of component 5 are introduced in this order into a powder mixer of adequate capacity, forcing them through a 15-mesh screen.

The remainder of components 1 and 5 and components 8, 9 and 10 are introduced in another powder mixer of adequate capacity, forcing them through a 30-mesh screen, mixing is them performed for 5-10 minutes.

Mixture "b" is added into the first mixer and mixing is performed for 10-15 minutes; component 7 is sprayed into the moving mass during the first 5 minutes, with the aid of an airbrush, adjusting the pressure of the propellant fluid between 0.1 and 0.5 atm.

Component 6 is added and mixing is continued for 5-10 minutes.

The previously prepared mixture is compacted directly with a rotary compacting machine equipped with molds of the required shapes and dimensions, at the intended weight.

The mixture thus obtained may be divided into single-or multiple-dose packages made of material impermeable to water vapor.

## 2 - Method of preparation in a non-conditioned environment

Direct mixing, compaction and packaging in a non-conditioned environment, so long as protected from the direct action of weather.

## Formula of the components

1 - Glycine sodium carbonate 7.5-60%
2 - Mannitol 5-20%
3 - Refined salt 10-55%
4 - Monoboric sodium citrate 7.5-60%
5 - Pulverized "Spirulina" alga 1-55%
6 - Polyethyleneglycol 6000 0.5-4%
7 - Scented essences 0.5-5%
8 - Potassium iodide 0.2-1%
9 - Tartrazine yellow dye (E102) 0.05%-0.5%
10 - Patent blue dye (E131) 0.001-0.01%

## Process for preparing the mixture to be compacted

Approximately 2/3 of component 1, components 2-3-4 and approximately 2/3 of component 5 are introduced in this order into a powder mixer of adequate capacity, forcing them through a 15-mesh screen.

The remainder of components 1 and 5 and components 8, 9 and 10 are introduced in that order in another powder mixer of adequate capacity, forcing them through a 30-mesh screen; mixing is then performed for 5-10'.

Mixture "b" is added into the first mixer and mixing is performed for 10-15 minutes; during the first 5 minutes, component 7 is sprayed into the moving mass with the aid of an airbrush, adjusting the pressure of the propellant fluid between 0.1 and 0.5 atm.

Component 6 is added and mixing is continued for 5-10 minutes.

The previously prepared mixture is directly compacted with a rotary compacting machine equipped with molds of the desired shapes and dimensions, at the intended weight and with a controlled-loading hopper.

The mixture thus obtained can be divided into single-or multiple-dose packages made of material impermeable to water vapor.

## Claims

1. Spontaneously water-dispersible composition, particularly suitable for treating the skin, characterized in that it comprises:
sodium chloride and
an effervescent agent.

2. Composition according to claim 1, characterized in that said effervescent agent is composed of sodium bicarbonate and tartaric acid.

3. Composition according to claim 1, characterized in that it comprises iodine and in that said iodine is preferably contained as iodide or iodate, and in that the weight ratio between said sodium chloride and said iodine is more preferably comprised between 10 and 100.

4. Composition according to claim 2, characterized in that the weight ratio between said sodium bicarbonate and said tartaric acid is comprised between 0.25 and 4.

5. Composition according to claim 1, characterized in that said effervescent agent comprises glycine sodium carbonate and monobasic sodium citrate.

6. Composition according to claim 5, characterized in that the weight ratio between said glycine sodium carbonate and said monobasic sodium citrate is comprised between 0.33 and 3.

7. Composition according to claim 1, characterized in that it furthermore comprises Spirulina alga.

8. Composition according to claim 7, characterized in that it said Spirulina alga is contained in a weight percentage comprised between 1% and 55%.

9. Composition according to claim 1, characterized in that it comprises metallic ions.

10. Composition according to claim 9, characterized in that said metallic ions comprise at least one of the following elements: iron, nickel, zinc, copper, manganese.

11. Composition according to claim 10, characterized in that said iron is contained in an amount comprised between 10 and 100 parts per million by weight in the finished product.

12. Composition according to claim 10, characterized in that said nickel is contained in an amount comprised between 10 and 100 parts per million by weight in the finished product.

13. Composition according to claim 10, characterized in that said zinc is contained in an amount comprised between 10 and 100 parts per million by weight in the finished product.

14. Composition according to claim 10, characterized in that said copper is contained in an amount comprised between 10 and 100 parts per million by weight in the finished product.

15. Composition according to claim 10, characterized in that said manganese is contained in an amount comprised between 1 and 50 parts per million by weight in the finished product.

16. Composition according to claim 1, characterized in that it comprises scenting essences in an amount comprised between 0.5% and 10% by weight in the finished product.

17. Composition according to claim 1, particularly suitable for preparing effervescent bath salts, in small granules, characterized in that it comprises the following composition, in which the percentages are given by weight with respect to the total:

1 - Sodium bicarbonate 5-60%
2 - Refined salt 10-55%
3 - Purified tartaric acid 5-60%
4 - Pulverized Spirulina alga 1-55%
5 - Gelatine 1-5%
6 - Glycerine 0.1-0.5%
7 - Scented essences 0.5-5%
8 - Potassium iodate 0.2-1%
9 - Tartrazine yellow dye (E102) 0.05-0.5%
10 - Patent blue dye (E131) 0.001-0.1%

18. Composition according to claim 1, particularly suitable for preparing effervescent bath salts, in medium and large grains, characterized in that it comprises the following composition, wherein the percentages are given by weight with respect to the total:

1 - Glycine sodium carbonate 7;.5-60%
2 - Refined salt 10-55%
3 - Monobasic sodium citrate 2.5-15%
4 - Pulverized "Spirulina" alga 1-55%
5 - Gum arabic 2-55%
6 - Scented essences 1-2%
7 - Potassium iodate 0.2-1%
8 - Tartrazine yellow dye (E102) 0.05%-0.5%
9 - Patent blue dye (E131) 0.001-0.01%

19. Composition according to claim 1, particularly adapted for preparing effervescent shower salts in paste form, characterized in that it comprises the following composition, in which the percentages are given by weight with respect to the total:

1 - Glycine sodium carbonate 7.5-60%
2 - Refined salt 10-55%
3 - Monobasic sodium citrate 7.5-60%
4 - Pulverized "Spirulina" alga 1-55%
5 - Scented essences 0.5-5%
6 - Potassium iodate 0.2-1%
7 - Tartrazine yellow dye (E102) 0.05%-0.5%
8 - Patent blue dye (E131) 0.001-0.01%
9 - Glycerine 10-30%

20. Composition according to claim 1, particularly suitable for preparing effervescent bath salts in tablets, characterized in that it comprises the following composition, wherein the percentages are given by weight with respect to the total:

1 - Sodium bicarbonate 5-60%
2 - Mannitol 5-20%
3 - Refined salt 10-35%
4 - Tartaric acid 5-60%
5 - Pulverized "Spirulina" alga 1-55%
6 - Polyethyleneglycol 6000 0.5-4%
7 - Scented essences 0.5-5%
8 - Potassium iodide 0.2-1%
9 - Tartrazine yellow dye (E102) 0.05%-0.5%
10 - Patent blue dye (E131) 0.001-0.01%

21. Composition according to claim 1, particularly suitable for preparing effervescent bath salts in tablets, characterized in that it comprises the following composition, wherein the percentages are given by weight with respect to the total:

1 - Glycine sodium carbonate 7.5-60%
2 - Mannitol 5-20%
3 - Refined salt 10-55%
4 - Monoboric sodium citrate 7.5-60%
5 - Pulverized "Spirulina" alga 1-55%
6 - Polyethyleneglycol 6000 0.5-4%
7 - Scented essences 0.5-5%
8 - Potassium iodide 0.2-1%
9 - Tartrazine yellow dye (E102) 0.05%-0.5%
10 - Patent blue dye (E131) 0.001-0.01%

22. Composition according to claim 1, particularly suitable for preparing effervescent bath salts, characterized in that it comprises the following composition, wherein the percentages are given by weight with respect to the total:

1 - Sodium bicarbonate 5-60%
2 - Refined salt 10-55%
3 - Tartaric acid 5-60%
4 - Pulverized "Spirulina" alga 1-55%
5 - Scented essences 0.5-5%
6 - Potassium iodide 0,2-1%
7 - Tartrazine yellow dye (E102) 0.05-0.5%
8 - Patent blue dye (E131) 0.001-0.01%

23. Use of a composition according to claim 1 to prepare bath salts.

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| X | CHEMICAL ABSTRACTS, vol. 93, no. 8, August 1980, page 506, abstract no. 79886v, Columbus, Ohio, US; & SU-A-727 196 (ALL-UNION SCIENTIFIC-RESEARCH INSTITUTE OF THE CHEMICAL INDUSTRY, PERM) 15-04-1980 * Abstract * | 1,2,4, 16 | A 61 K 7/50 |
| X | PATENT ABSTRACTS OF JAPAN, vol. 6, no. 131 (C-114)[1009], 17th July 1982; & JP-A-57 56 420 (SHISEIDO K.K.) 05-04-1982 * Abstract * | 1,2 | |
| X | PATENT ABSTRACTS OF JAPAN, vol. 11, no 361 (C-459)[2808], 25th November 1987; & JP-A-62 132 816 (LION CORP.) 16-06-1987 * Abstract * | 1,2,9, 10 | |
| A | FR-A-2 555 444 (J. AUDY-ROWLAND) * Claims * | 7,8 | TECHNICAL FIELDS SEARCHED (Int. Cl.5) A 61 K |

The present search report has been drawn up for all claims

| Place of search | Date of completion of the search | Examiner |
|---|---|---|
| THE HAGUE | 08-11-1989 | WILLEKENS G.E.J. |

EPO FORM 1503 03.82 (P0401)